# EUROPEAN PATENT APPLICATION

(11) **EP 4 733 822 A2**
(43) Date of publication of application: **29.04.2026**
(21) Application number: 25207418.2
(22) Date of filing: 08.10.2025
(51) Int. Cl.: G02B 6/38

(54) **HYBRID CONNECTOR WITH INTEGRATED OPTICAL AND ELECTRICAL TERMINATION**

(30) Priority: 23.10.2024 US 202418924131
(71) Applicant: Winchester Interconnect Micro LLC, Orange, CA 92865 (US)
(72) Inventor: ASHOK, Pradeep Kumar, Orange, CA (US)
(74) Representative: Bardehle Pagenberg Partnerschaft mbB Patentanwälte Rechtsanwälte

(57) **Abstract**

This document discloses a hybrid connector assembly (102, 104) designed to terminate an optical conductor and multiple electrical conductors. The connectors (102, 104) includes a set of electrical contacts arranged to terminate the electrical conductors, with each contact longitudinally offset from the others and encircling the optical conductor. An electrical insulator (112, 314) is employed to secure and isolate the electrical contacts within the connector. This innovative design allows for the efficient and reliable termination of both optical and electrical connections within a single compact and well-organized connector (102, 104).

## Description

The subject matter disclosed herein relates to cable connectors and, in particular, to hybrid connectors with integrated optical and electrical termination.

Attaching a hybrid connector to an end of an elongate device having both optical and electrical conductors may require the connector to have a larger outer diameter than the elongate device itself, depending on the number of interconnect termination points required for power source and other electrical signals. For applications such as catheters for peripheral vascular access device (PVAD) deliveries, this increase in outer diameter of the connector necessitates the use of a special splitable sheath over the catheter. However, this approach often encounters problems such as premature splitting and/or ovalizing of the sheath of the catheter.

Previous approaches to terminating optical conductors and electrical conductors in a single connector have typically involved separate connectors for each type of conductor, resulting in a bulky and complex assembly. In these conventional designs, the optical conductor and electrical conductors are terminated in separate compartments within the connector, requiring additional components for insulation and securing of the conductors. The optical conductor termination often involves specialized connectors and tools, adding to the complexity and cost of the overall system.

Efforts to integrate optical and electrical connections in a single connector have been made by incorporating optical fibers within electrical connectors or by using hybrid connectors with separate compartments for optical and electrical terminations. However, these approaches have limitations in terms of size, ease of assembly, and overall performance. The optical fibers in these hybrid connectors are typically positioned in close proximity to the electrical contacts, leading to potential interference and signal degradation.

In view of the challenges associated with existing hybrid connectors for terminating optical and electrical conductors, there remains a need for a more efficient and compact solution that provides reliable termination of both types of conductors within a single connector. However, none of these approaches have provided a comprehensive solution that combines the features described in this disclosure.

In some aspects, the techniques described herein relate to a hybrid plug connector configured to terminate an optical conductor and a plurality of electrical conductors. The hybrid plug connector includes a plurality of electrical contacts configured to terminate the plurality of electrical conductors. The electrical contacts are longitudinally offset from one another and are configured to at least partially radially surround the optical conductor. The hybrid plug connector also includes an electrical insulator securing and separating the plurality of electrical contacts.

In some aspects, the techniques described herein relate to a hybrid receptacle connector configured to terminate an optical conductor and a plurality of electrical conductors. The hybrid receptacle connector includes a plurality of electrical contacts configured to terminate the plurality of electrical conductors. The electrical contacts are longitudinally offset from one another and are configured to semi-radially surround and electrically interface with a corresponding plurality of electrical contacts in a hybrid plug connector. The hybrid receptacle connector further includes an electrical insulator securing and separating the plurality of electrical contacts and configured to receive at least a portion of the hybrid plug connector.
**FIG. 1** is an isometric view of a hybrid connector assembly according to some embodiments.
**FIG. 2** is an isometric view of a plug connector of the hybrid connector assembly of **FIG. 1** according to some embodiments.
**FIG. 3** is an isometric view of a receptacle connector for the plug connector of **FIG. 2** in the hybrid connector assembly of FIG. 1 according to some embodiments.
**FIG. 4** is an isometric cross-section view of the hybrid connector of **FIG. 2** mated with the receptacle connector of FIG. 3 according to some embodiments.
**FIG. 5** is a side cross-section view of the hybrid connector of **FIG. 2** and the receptacle connector of **FIG. 3** in a mated and locked condition according to some embodiments.
**FIG. 6** is a side cross-section view of the plug connector of **FIG. 2** and the receptacle connector of **FIG. 3** in a mated and unlocked condition according to some embodiments.
**FIG. 7** is a side cross-section view of the plug connector of **FIG. 2** and the receptacle connector of **FIG. 3** in a partially mated and unlocked condition according to some embodiments.

The present disclosure describes a hybrid connector with integrated optical and electrical termination for an elongate hybrid device having both optical and electrical conductors.

To avoid the problems described above, a low-profile hybrid connector assembly having a hybrid plug connector with an outer diameter not exceeding that of the elongate hybrid device is desired. This is particularly beneficial in catheter applications because it allows an access sheath to be easily removed from the catheter without the need for splitting it.

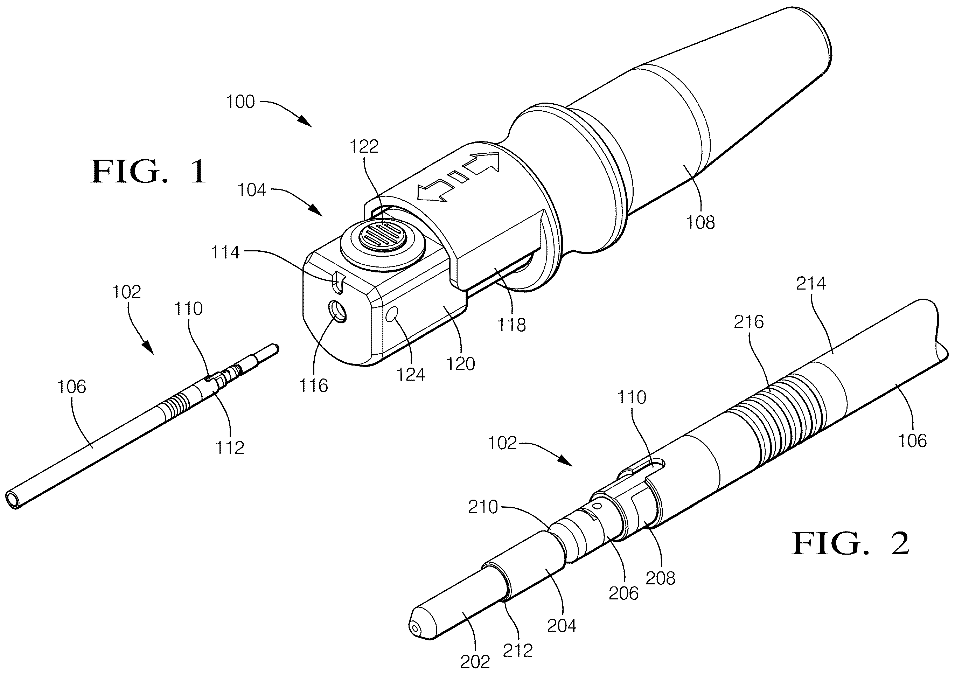

A low-profile hybrid connector assembly, suitable for use with elongate hybrid device, such as a catheter, is shown in **FIG. 1****.** This hybrid connector connects multiple electrical conductors providing electrical power and/or signals to electrical devices, such as a pump, and fiber optic conductors for transmitting data.

A non-limiting example of a hybrid connector system 100, as shown in **FIG. 1****,** features a hybrid plug connector 102. The hybrid connector system also includes a corresponding hybrid receptacle connector 104. This hybrid connector system 100 minimizes a diameter required for the hybrid plug connector 102. In this illustrated example the hybrid plug connector 102 is configured to terminate a catheter 106 having an optical conductor and a plurality of electrical conductors and the mating hybrid receptacle connector 104 is configured to terminate a cable 108 having a corresponding optical conductor and a plurality of corresponding electrical conductors.

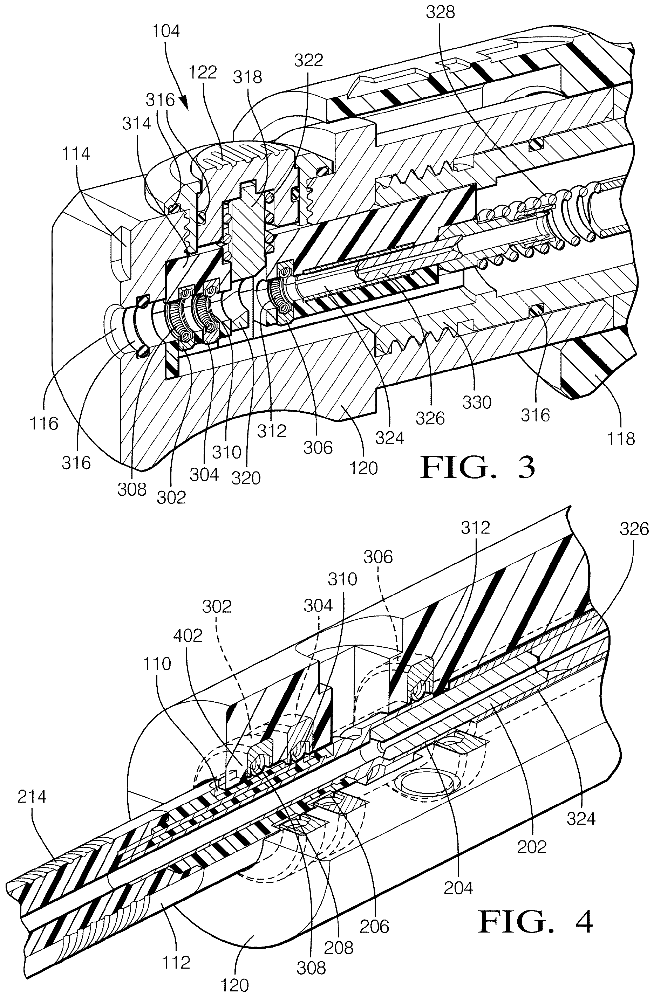

To mate the hybrid plug connector 102 with the hybrid receptacle connector 104, the user locates the key seat groove 110 in an insulator 112 of the hybrid plug connector 102 and aligns it with the corresponding key indicator 114 on the hybrid receptacle connector 104 and pushes the hybrid plug connector into the mating interface 116 until it locks into place. The key seat groove 110 can be seen in greater detail in **FIG. 3****.** To disengage the hybrid plug connector 102 from the hybrid receptacle connector 104, the user slides the safety sleeve 118 back onto the hybrid receptacle connector 104 until it latches into rearward divots (not shown, but similar to forward divots 124) in the housing 120, thereby exposing a lock actuation button 122. The user then pushes the lock actuation button 122, pulls the hybrid plug connector 102 out of the hybrid receptacle connector 104, and slides the safety sleeve 118 forward until it snaps into place in forward divots 124 on the housing 120, thereby covering the lock actuation button 122.

**FIG. 2** shows the overall layout of the hybrid plug connector 102 having an inline coaxially mounted ceramic fiber optic ferrule 202 and sequentially stacked gold-plated plug contacts 204, 206, 208 that are held together by the injection-molded plastic insulator 112. The hybrid plug connector 102 in this example terminates three electrical conductors with the plug contacts 204, 206, 208 and one fiber optic conductor in the fiber optic ferrule 202.

As shown in the non-limiting example of **FIG. 2****,** the first plug contact 204 is screw-machined from a brass material and then gold plated. The first plug contact 204 also features a machined locking groove 210. The second and third plug contacts 206, 208 are stamped from 127 µm (0.005 in.) thick beryllium copper alloy and are also gold plated. In another embodiments, the plug contacts may be formed of different materials by different process and be unplated or plated with alternative materials. A fiber optic ferrule 202 is press fit into a bore 212 at an end of first plug contact 204 and is configured to receive and terminate a fiber optic cable. In alternative embodiments, the fiber optic ferrule may be formed of other suitable materials, have a different diameter, and/or secured to the first plug contact by other means. The first, second, and third plug contacts 204, 206, 208 are held together in the insert-molded insulator 112 which is formed of a dielectric material, such as 40% glass filled (GF) liquid crystal polymer (LCP) or polyphenylene sulfide (PPS). This insulator 112 also defines the key seat groove 110 which is formed during the insert-molding process. A plastic strain relief 214 is then over-molded to connect the insulator 112 to the catheter's sheath 106. The strain relief 214 also has grooved features 216 on its outer surface to facilitate the user's grip.

**FIG. 3** shows a cross-section of the hybrid receptacle connector 104, detailing a lock actuation mechanism and the mating electrical contact arrangement. that features brass rings 302, 304, 306 each holding a nickel/gold plated toroidal spring 308, 310, 312. The brass rings 302, 304, 306 are spaced inside a plastic insulator 314, thereby providing 360° radial electrical contacts. The brass rings 302, 304, 306 are machined and gold-plated and each have an internal groove. Each internal groove accommodates one of the toroidal springs 308, 310, 312. The toroidal spring 308, 310, 312 allow these 360° radial electrical contacts to have varying inner diameters depending on the corresponding plug contact's outer diameter.

The insulator 314 is a keyed split-insulator assembly that is formed of a dielectric material, such as 40% GF LCP or PPS. The insulator 314 holds the brass rings 302, 304, 306 and spaces the brass rings 302, 304, 306 precisely to make contact between the toroidal spring 308, 310, 312 and the plug contacts 204, 206, 208 when the hybrid plug connector 102 and hybrid receptacle connector 104 are mated. Additionally, environmental seals or O-rings 316 may be used to protect the internal components of the hybrid receptacle connector 104 from environmental contaminants during use.

The locking mechanism includes a lock shaft 318 that has a 25° angled lock wedge 320 designed to glide over the hybrid plug connector's body and self-lock into the locking groove 210 in first plug contact 204. A compression spring 322 keeps the lock shaft 318 at constant tension for self-locking capability within the locking groove 210 until released by pressing the lock actuation button 122.

The insulator 314 also contains a fiber optic split-sleeve 324, and a spring loaded and keyed fiber optic ferrule assembly 326 configured to receive and terminate a corresponding fiber optic cable and hold the ferrule assembly 326 in contact with the fiber optic ferrule 202 of the hybrid plug connector 102. The split-sleeve 324, spring 328, and ferrule assembly 326 are encased between the housing 120 and a threaded shaft 330 (both typically made of nickel-plated aluminum). The back end of the threaded shaft 330 is then over-molded with a soft plastic to hold hybrid receptacle connector 104 and to provide strain-relief for the cables.

**FIG. 4** shows the contact engagement between the plug contacts 204, 206, 208 of the hybrid plug connector 102 and the toroidal springs 308, 310, 312 of the hybrid receptable connector 104 in detail. The toroidal springs 308, 310, 312 wrap around the plug contacts 204, 206, 208 upon mating, making a consistent 360° connection for an electrical connection. **FIG. 4** also shows the engagement between a key feature 402 defined by the ring 302 and the key seat groove 110 that is used for proper alignment of the fiber optic ferrules 202, 326.

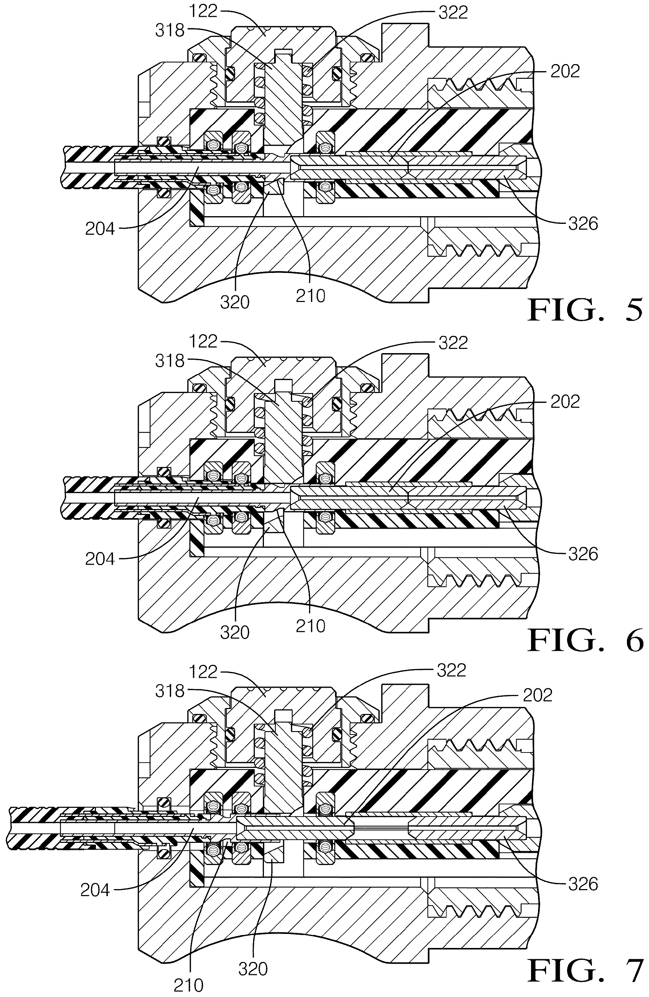

**FIG. 5** shows the lock wedge 320 of the lock shaft 318 engaged with the disengagement on the locking groove 210 of first plug contact 206. **FIG. 6** shows the lock actuation button 122 in a depressed position, thereby compressing the compression spring 322 and moving the lock wedge 320 out of engagement with the locking groove 210. The lock actuation button 122 travels approximately 380 µm (0.015 in) to unlock the first plug contact, thereby enabling the removal of the hybrid plug connector 102 from the hybrid receptacle connector 104 as shown in **FIG. 7****.**

### Discussion of Possible Embodiments

The following are non-exclusive descriptions of possible embodiments of the present invention.

In some aspects, the techniques described herein relate to a hybrid plug connector configured to terminate an optical conductor and a plurality of electrical conductors. The hybrid plug connector includes a plurality of electrical contacts configured to terminate the plurality of electrical conductors. The electrical contacts are longitudinally offset from one another and are configured to at least partially radially surround the optical conductor. The hybrid plug connector also includes an electrical insulator securing and separating the plurality of electrical contacts.

The hybrid plug connector of the preceding paragraph can optionally include, additionally and/or alternatively any, one or more of the following features/steps, configurations and/or additional components.

In some aspects, the techniques described herein relate to a hybrid plug connector, further including a ferrule configured to receive and terminate the optical conductor, wherein the ferrule is disposed in a first electrical contact in the plurality of electrical contacts.

In some aspects, the techniques described herein relate to a hybrid plug connector, wherein the ferrule and the first electrical contact are concentric with the optical conductor and wherein the ferrule is in a friction fit within the first electrical contact.

In some aspects, the techniques described herein relate to a hybrid plug connector, wherein the first electrical contact defines a groove configured to receive a latch of a corresponding hybrid receptacle connector, the groove and the latch being configured to secure the hybrid plug connector to the corresponding hybrid receptacle connector.

In some aspects, the techniques described herein relate to a hybrid plug connector, the electrical insulator defining a key seat groove configured to receive a key feature of a corresponding hybrid receptacle connector, the key seat groove and the key feature being configured to cooperate to align the ferrule with a corresponding ferrule that is configured to receive and terminate another optical conductor in the corresponding hybrid receptacle connector.

In some aspects, the techniques described herein relate to a hybrid plug connector, wherein at least one electrical contact in the plurality of electrical contacts is configured to radially surround the optical conductor.

In some aspects, the techniques described herein relate to a hybrid plug connector, wherein at least one electrical contact in the plurality of electrical contacts has a different radial distance from the optical conductor than other electrical contacts in the plurality of electrical contacts.

In some aspects, the techniques described herein relate to a hybrid plug connector, the electrical insulator including a glass filled polymer material and being overmolded onto the plurality of electrical contacts.

In some aspects, the techniques described herein relate to a hybrid plug connector, the hybrid plug connector is configured to terminate a catheter including the optical conductor and the plurality of electrical conductors.

In some aspects, the techniques described herein relate to a hybrid plug connector, further including a flexible strain relief configured to attach the hybrid plug connector to the catheter.

In some aspects, the techniques described herein relate to a catheter, including an optical conductor, a plurality of electrical conductors, and the hybrid plug connector according to any of the preceding paragraphs terminating the optical conductor and the plurality of electrical conductors.

In some aspects, the techniques described herein relate to a catheter according to the previous paragraph, further including a flexible strain relief attaching the hybrid plug connector to the sheath.

In some aspects, the techniques described herein relate to a hybrid receptacle connector configured to terminate an optical conductor and a plurality of electrical conductors. The hybrid receptacle connector includes a plurality of electrical contacts configured to terminate the plurality of electrical conductors. The electrical contacts are longitudinally offset from one another and are configured to semi-radially surround and electrically interface with a corresponding plurality of electrical contacts in a hybrid plug connector. The hybrid receptacle connector further includes an electrical insulator securing and separating the plurality of electrical contacts and configured to receive at least a portion of the hybrid plug connector.

The hybrid receptacle connector of the preceding paragraph can optionally include, additionally and/or alternatively any, one or more of the following features/steps, configurations and/or additional components.

In some aspects, the techniques described herein relate to a hybrid receptacle connector, the electrical insulator defining a key feature configured to be received in a key seat groove of a corresponding hybrid plug connector, the key seat groove and the key feature being configured to cooperate to align a ferrule in the hybrid receptacle connector that is configured to receive and terminate the optical conductor with a corresponding ferrule that is configured to receive and terminate another optical conductor in the corresponding hybrid plug connector.

In some aspects, the techniques described herein relate to a hybrid receptacle connector, wherein the plurality of electrical contacts include electrically conductive toroidal springs.

In some aspects, the techniques described herein relate to a hybrid receptacle connector, wherein the plurality of electrical contacts further include electrically conductive rings in which the toroidal springs are disposed.

In some aspects, the techniques described herein relate to a hybrid receptacle connector, wherein one toroidal spring in the plurality of electrical contacts has a larger diameter than another toroidal spring in the plurality of electrical contacts.

In some aspects, the techniques described herein relate to a hybrid receptacle connector, further including a housing containing the electrical insulator; and a releasable latch disposed within the housing that is configured to engage a groove in a corresponding hybrid plug connector, the groove and the latch configured to secure the hybrid receptacle connector to the corresponding hybrid plug connector.

In some aspects, the techniques described herein relate to a hybrid receptacle connector, further including: a lock shaft defining the latch; and a compression spring configured to maintain the latch within the groove until a force applied to the lock shaft overcomes a spring force of the compression spring and moves the latch out of the groove.

In some aspects, the techniques described herein relate to a hybrid receptacle connector, further including an actuation button configured to move the lock shaft; and a cover slidably attached to the housing and configured to conceal the actuation button when in a first position and expose the actuation button when moved to a second position.

While the invention has been described with reference to an exemplary embodiment(s), it will be understood by those skilled in the art that various changes may be made, and equivalents may be substituted for elements thereof without departing from the scope of the invention. In addition, many modifications may be made to adapt a particular situation or material to the teachings of the invention without departing from the essential scope thereof. Therefore, it is intended that the invention is not limited to the disclosed embodiment(s), but that the invention will include all embodiments falling within the scope of the appended claims.

As used herein, 'one or more' includes a function being performed by one element, a function being performed by more than one element, e.g., in a distributed fashion, several functions being performed by one element, several functions being performed by several elements, or any combination of the above.

It will also be understood that, although the terms first, second, etc. are, in some instances, used herein to describe various elements, these elements should not be limited by these terms. These terms are only used to distinguish one element from another. For example, a first contact could be termed a second contact, and, similarly, a second contact could be termed a first contact, without departing from the scope of the various described embodiments. The first contact and the second contact are both contacts, but they are not the same contact.

The terminology used in the description of the various described embodiments herein is for the purpose of describing particular embodiments only and is not intended to be limiting. As used in the description of the various described embodiments and the appended claims, the singular forms "a", "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "includes," "including," "comprises," and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" is, optionally, construed to mean "when" or "upon" or "in response to determining" or "in response to detecting," depending on the context. Similarly, the phrase "if it is determined" or "if [a stated condition or event] is detected" is, optionally, construed to mean "upon determining" or "in response to determining" or "upon detecting [the stated condition or event]" or "in response to detecting [the stated condition or event]," depending on the context.

Additionally, while terms of ordinance or orientation may be used herein these elements should not be limited by these terms. All terms of ordinance or orientation, unless stated otherwise, are used for purposes distinguishing one element from another, and do not denote any particular order, order of operations, direction or orientation unless stated otherwise.

## Claims

1. A hybrid plug connector (102) configured to terminate an optical conductor and a plurality of electrical conductors, said hybrid plug connector (102) comprising:
a plurality of electrical contacts configured to terminate the plurality of electrical conductors, the plurality of electrical contacts being longitudinally offset from one another and configured to at least partially radially surround the optical conductor; and
an electrical insulator (112) securing and separating the plurality of electrical contacts.

2. The hybrid plug connector (102) in accordance with claim 1, further comprising a ferrule configured to receive and terminate the optical conductor, wherein the ferrule is disposed in a first electrical contact in the plurality of electrical contacts.

3. The hybrid plug connector (102) in accordance with claim 2, wherein the ferrule and the first electrical contact are concentric with the optical conductor and wherein the ferrule is in a friction fit within the first electrical contact.

4. The hybrid plug connector (102) in accordance with claim 2 or 3, wherein the first electrical contact defines a groove configured to receive a latch of a corresponding hybrid receptacle connector (104), the groove and the latch being configured to secure the hybrid plug connector (102) to the corresponding hybrid receptacle connector (104).

5. The hybrid plug connector (102) in accordance with any one of the preceding claims 2 to 4, the electrical insulator (112) defining a key seat groove (110) configured to receive a key feature (402) of a corresponding hybrid receptacle connector (104), the key seat groove (110) and the key feature (402) being configured to cooperate to align the ferrule with a corresponding ferrule that is configured to receive and terminate another optical conductor in the corresponding hybrid receptacle connector (104).

6. The hybrid plug connector (102) in accordance with any one of the preceding claims, wherein at least one electrical contact in the plurality of electrical contacts is configured to radially surround the optical conductor.

7. The hybrid plug connector (102) in accordance with any one of the preceding claims, wherein at least one electrical contact in the plurality of electrical contacts has a different radial distance from the optical conductor than other electrical contacts in the plurality of electrical contacts.

8. A catheter (106), comprising:
an optical conductor and a plurality of electrical conductors;
a sheath in which the optical conductor and the plurality of electrical conductors is disposed; and
the hybrid plug connector (102) in accordance with claim 1 terminating the optical conductor and the plurality of electrical conductors.

9. A hybrid receptacle connector (104) configured to terminate an optical conductor and a plurality of electrical conductors, said hybrid receptacle connector (104) comprising:
a plurality of electrical contacts configured to terminate the plurality of electrical conductors, the plurality of electrical contacts being longitudinally offset from one another and configured to semi-radially surround and electrically interface with a corresponding plurality of electrical contacts in a hybrid plug connector (102); and
an electrical insulator (314) securing and separating the plurality of electrical contacts and configured to receive at least a portion of the hybrid plug connector (102).

10. The hybrid receptacle connector (104) in accordance with claim 9, the electrical insulator (314) defining a key feature (402) configured to be received in a key seat groove (110) of a corresponding hybrid plug connector (102), the key seat groove (110) and the key feature (402) being configured to cooperate to align a ferrule in the hybrid receptacle connector (104) that is configured to receive and terminate the optical conductor with a corresponding ferrule that is configured to receive and terminate another optical conductor in the corresponding hybrid plug connector (102).

11. The hybrid receptacle connector (104) in accordance with claim 9 or 10, wherein the plurality of electrical contacts comprise electrically conductive toroidal springs (308, 310, 312).

12. The hybrid receptacle connector (104) in accordance with claim 11, wherein the plurality of electrical contacts further comprise electrically conductive rings in which the toroidal springs (308, 310, 312) are disposed, preferably one toroidal spring (308) in the plurality of electrical contacts has a larger diameter than another toroidal spring (310, 312) in the plurality of electrical contacts.

13. The hybrid receptacle connector (104) in accordance with any one of claims 10 to 12, further comprising:
a housing (120) containing the electrical insulator (314); and
a releasable latch disposed within the housing (120) that is configured to engage a groove in a corresponding hybrid plug connector (102), the groove and the latch configured to secure the hybrid receptacle connector (104) to the corresponding hybrid plug connector (102).

14. The hybrid receptacle connector (104) in accordance with claim 13, further comprising:
a lock shaft (318) defining the latch; and
a compression spring (322) configured to maintain the latch within the groove until a force applied to the lock shaft (318) overcomes a spring force of the compression spring (322) and moves the latch out of the groove.

15. The hybrid receptacle connector (104) in accordance with claim 14, further comprising:
an actuation button configured to move the lock shaft (318); and
a cover slidably attached to the housing (120) and configured to conceal the actuation button when in a first position and expose the actuation button when moved to a second position.
